# EUROPÄISCHE PATENTANMELDUNG

(11) **EP 3 545 988 A1**
(43) Veröffentlichungstag der Anmeldung: **02.10.2019**
(21) Anmeldenummer: 18165186.0
(22) Anmeldetag: 29.03.2018
(51) Int. Cl.: A61M 5/20, A61M 5/24, A61M 5/315

(54) **DOSIERVORRICHTUNG MIT STELLEINRICHTUNG FÜR EINEN INJEKTOR**

(71) Anmelder: Tecpharma Licensing AG, 3401 Burgdorf (CH)
(72) Erfinder: Hirschel, Jürg, 3007 Bern (CH)

(57) **Zusammenfassung**

Die Erfindung betrifft eine Dosiervorrichtung für einen Injektor (1) zur dosierten Abgabe einer medizinischen Substanz umfassend einen Antrieb, ein durch den Antrieb bewegbares Ausschüttelement zum Ausschütten der Substanz aus dem Injektor (1) und eine Stelleinrichtung. Die Stelleinrichtung weist eine Freigabestellung auf, in welcher der Antrieb durch die Stelleinrichtung freigegeben ist und der Antrieb das Ausschüttelement bewegen kann. Weiter weist die Stelleinrichtung eine Sperrstellung aufweist, in welcher der Antrieb durch die Stelleinrichtung mittels Formschluss blockiert ist, wodurch der Antrieb das Ausschüttelement nicht bewegen kann. Dabei ist in der Freigabestellung die Stelleinrichtung mit dem Antrieb gekoppelt, so dass eine Bewegung der Stelleinrichtung auf den Antrieb oder eine Bewegung des Antriebs auf die Stelleinrichtung übertragen wird. Weiter betrifft die Erfindung einen Injektor (1) mit einer solchen Dosiervorrichtung.

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf eine Dosiervorrichtung mit einer Stelleinrichtung und auf einen Injektor mit einer solchen Dosiervorrichtung. Die Stelleinrichtung ist zwischen einer Freigabestellung, in welcher ein Antrieb zum Ausschütten der Substanz freigegeben ist, und einer Sperrstellung, in welcher der Antrieb blockiert ist und somit keine Ausschüttung erfolgen kann, verstellbar.

### HINTERGRUND DER ERFINDUNG

Aus dem Stand der Technik sind unterschiedliche Injektionsgeräte bekannt. Einige Injektionsgeräte wurden weiterentwickelt, um die Handhabung für den Benutzer zu vereinfachen. Insbesondere für Benutzer mit eingeschränkten Bewegungsmöglichkeiten, zittrigen Händen, Muskelsteifheit oder Muskelschwäche können sich Probleme bei der Bedienung des Injektors ergeben. Daher wurden Injektoren mit einstellbarer Dosis entwickelt, bei denen die Kraft zum Ausschütten der medizinischen Substanz aus dem Injektor nicht durch den Benutzer erbracht werden muss, sondern die einen elektrischen Antrieb für das Ausschütten der Substanz umfassen.

Einen solchen Injektor mit einem Elektromotor beschreibt beispielsweise die Patentanmeldung WO 2011/146713 A1. Die Schrift offenbart ein Injektionssystem mit einem Injektor und einer separaten Steuereinheit, wobei die Steuereinheit drahtlos mit dem Injektor kommunizieren kann. Die Steuereinheit umfasst einen Touchscreen für die Benutzereingaben wie das Einstellen der Injektionsgeschwindigkeit, der Dosis oder anderen Injektionsparameter. Der Injektor wird am Handgelenk des Benutzers befestigt. Mittels eines Injektionsknopfs am Injektor kann der Benutzer den Injektionsvorgang starten und beenden. Der Elektromotor im Injektor bewegt eine Kolbenstange, wenn der Injektionsknopf betätigt wird, um die flüssige Substanz aus einer Kartusche im Injektor auszusch ütten.

Einen anderen automatisierten Injektor offenbart die Patentanmeldung WO 2015/138093 A1, indem diese eine automatische Dosiereinrichtung, welche auf einen herkömmlichen Injektionspen aufsetzbar ist, beschreibt. Die Dosiereinrichtung umfasst einen oberen Teil und einen unteren Teil. Der untere Teil beinhaltet eine Steuerung, eine Empfangseinheit, Sensoren und eine Antriebseinheit mit einem Piezo-Schrittmotor. Im montierten Zustand ist dieser untere Teil der Dosiereinrichtung unbeweglich mit dem zylinderförmigen Pen-Gehäuse verbunden, während der obere Teil einen Aufsatz umfasst, der rotationsfest mit dem Dosiseinstellknopf des Pens verbunden ist. Die Steuerung erhält von einem Messsystem oder einem Datenmanagementsystem auf einem Mobiltelefon oder PC drahtlos Informationen über eine zu verabreichende Dosis. Mittels der Antriebseinheit und dem Aufsatz stellt die Steuerung basierend auf den empfangenen Daten die gewünschte Dosis am Pen ein ohne dass der Benutzer am Dosiseinstellknopf drehen muss. Anschliessend kann der Benutzer durch Betätigen des Injektionsknopfes die Injektion beginnen. Zudem erkennt die Steuerung wenn der Dosiseinstellknopf manuell durch den Benutzer eingestellt oder korrigiert wurde. Ferner kann die Steuerung Informationen über die verabreichte Dosis an das Datenmanagementsystem senden.

Solche bekannten Injektionsgeräte mit einem elektrischen Antrieb oder einer elektrischen Einstellmöglichkeit haben den Nachteil, dass bei einer Fehlfunktion möglicherweise die Substanz nicht korrekt oder nicht in der richtigen Dosis ausgeschüttet wird. Stoppt beispielsweise der Antrieb nicht rechtzeitig, wird zu viel ausgeschüttet und es kommt zu einer Überdosierung. Das kann schwerwiegende Folgen für den Benutzer haben.

### DARSTELLUNG DER ERFINDUNG

Es ist eine Aufgabe der Erfindung, für einen Benutzer mit eingeschränkten Bewegungsmöglichkeiten eine sichere und zuverlässige Ausschüttung der dosierten Substanz zu ermöglichen.

Diese Aufgabe wird gelöst durch eine Dosiervorrichtung für einen Injektor zum portionenweisen Ausschütten einer eingestellten Dosis einer Substanz, wobei während dem Ausschütten der Antrieb mit der Stelleinrichtung gekoppelt ist gemäss dem unabhängigen Anspruch 1. Weiter umfasst die Erfindung einen Injektor mit einer solchen Dosiervorrichtung. Bevorzugte Ausführungsformen sind Gegenstand der abhängigen Ansprüche.

Gemäss der Erfindung umfasst die Dosiervorrichtung einen Antrieb, ein durch den Antrieb bewegbares Ausschüttelement zum Ausschütten der Substanz aus dem Injektor und eine Stelleinrichtung. Diese Stelleinrichtung weist
a) eine Freigabestellung auf, in welcher der Antrieb durch die Stelleinrichtung freigegeben ist und der Antrieb das Ausschüttelement bewegen kann und weist
b) eine Sperrstellung auf, in welcher der Antrieb durch die Stelleinrichtung mittels Formschluss blockiert ist, wodurch der Antrieb das Ausschüttelement nicht bewegen kann.

Dabei ist in der Freigabestellung die Stelleinrichtung mit dem Antrieb gekoppelt, so dass eine Bewegung der Stelleinrichtung auf den Antrieb oder eine Bewegung des Antriebs auf die Stelleinrichtung übertragen wird. Es kann somit beispielsweise eine Bewegung zuerst von der Stelleinrichtung auf den Antrieb und anschliessend eine Bewegung vom Antrieb auf die Stelleinrichtung übertragen werden.

Diese Kopplung zwischen der Stelleinrichtung und dem Antrieb bewirkt, dass in der Freigabestellung zwischen der Stelleinrichtung und dem Antrieb eine kinematische Kette besteht, wodurch Bewegungen in beide Richtungen übertragen werden. Die Kopplung kann auch als Zwangsführung bezeichnet werden, da sie zwingend eine Bewegung des Antriebs auf die Stelleinrichtung und umgekehrt eine Bewegung der Stelleinrichtung auf den Antrieb überträgt.

Die Kopplung in der Freigabestellung kann als feste Kupplung, Kulissensteuerung oder als Getriebeverbindung mit einem definierten Übersetzungsverhältnis ausgebildet sein. Dabei muss zwischen Stelleinrichtung und Antrieb nicht zwingend eine Über- oder Untersetzung vorliegen, sondern das Übersetzungsverhältnis kann beispielsweise auch den Wert 1:1 aufweisen. Ferner muss das Übersetzungsverhältnis in der Freigabestellung nicht zwingend konstant sein, sondern kann sich ändern.

Die erwähnte Kopplung zwischen Stelleinrichtung und Antrieb besteht vorzugsweise nur in der Freigabestellung und ist in der Sperrstellung aufgehoben. Das bedeutet, im Gegensatz zur Freigabestellung ist in der Sperrstellung beispielsweise eine Bewegung der Stelleinrichtung nicht auf den Antrieb übertragbar. Die Stelleinrichtung ist also vorzugsweise in der Sperrstellung bewegbar ohne dass sich der Antrieb bewegt. Während der Zeit, in der sich die Stelleinrichtung in der Freigabestellung befindet, kann das durch den Antrieb bewegte Ausschüttelement die Substanz aus dem Injektor ausschütten. Vorzugsweise wird dabei das Ausschüttelement in eine im Injektor befindliche Kartusche hineinbewegt, wodurch die Substanz aus der Kartusche gestossen wird.

Dabei bedeutet der Begriff "Freigabestellung" nicht, dass der Antrieb oder die Stelleinrichtung in der Freigabestellung eine einzige physische Stellung oder Position einnehmen müssen. So können in der Freigabestellung der Antrieb und die Stelleinrichtung oder Elemente der Stelleinrichtung mehrere Positionen einnehmen. Vorzugsweise sind in der Freigabestellung der Antrieb und Elemente der Stelleinrichtung in einer oder mehreren Zonen oder über einen oder mehrere Bereiche, insbesondere Drehwinkelbereiche bewegbar.

Da die Stelleinrichtung während dem Ausschütten der eingestellten Dosis vorzugsweise mehrmals von der Sperrstellung in die Freigabestellung und wieder in die Sperrstellung verstellbar ist, kann die Ausschüttung der Dosis stückweise bzw. portionenweise erfolgen. Der Antrieb wird jeweils durch die Stelleinrichtung zeitweise freigegeben, um eine Teildosis der zu verabreichenden Dosis auszuschütten. Anschliessend wird der Antrieb wieder blockiert. Die Anzahl Verstellungen von der Sperrstellung zur Freigabestellung und wieder in die Sperrstellung richtet sich nach der Höhe der gewählten Dosis bzw. nach der Zeitdauer in der sich die Stelleinrichtung in der Freigabestellung befindet. Das bedeutet, im Unterschied zu bekannten Injektionsgeräten, muss mit der erfindungsgemässen Dosiervorrichtung die Ausschüttung nicht in einer singulären, ununterbrochenen Bewegung oder einem einzigen Hub erfolgen, sondern kann portionenweise in mehreren Teildosen erfolgen.

Die erfindungsgemässe Dosiervorrichtung mit der Stelleinrichtung ermöglicht ein exaktes, kontrolliertes und sicheres Ausschütten, da die eingestellte Dosis nur portionenweise in Teildosen ausgeschüttet werden kann. Die Ausschüttung kann dadurch bei Bedarf während dem Ausschüttvorgang unterbrochen werden. Zudem besteht nicht wie bei bekannten Dosiervorrichtungen, bei denen die eingestellte Dosis in einer ununterbrochenen Bewegung ausgeschüttet wird und die nur einen Endanschlag aufweisen, die Gefahr, dass die Ausschüttung durch den Endanschlag nicht gestoppt wird und somit eine Überdosierung erfolgt. Die Dosiervorrichtung gemäss der Erfindung benötigt keinen Endanschlag für den Hub zum Ausschütten, da bei einer grösseren Dosis der Antrieb immer wieder durch die Stelleinrichtung blockiert wird während dem Ausschüttvorgang. Durch diese portionenweise Ausschüttung in Teildosen ist der Ausschüttvorgang besser überwachbar und kontrollierbar. Zudem ist bei der erfindungsgemässen Dosiervorrichtung in der Freigabestellung eine Bewegung der Stelleinrichtung an eine Bewegung des Antriebs gekoppelt und umgekehrt eine Bewegung des Antriebs an eine Bewegung der Stelleinrichtung gekoppelt. Dadurch ist die Bewegung des Antriebs gut kontrollierbar, so dass ein präzises und kontrolliertes Ausschütten ermöglicht wird.

Trotz dieser portionenweisen Ausschüttung kann mit der erfindungsgemässen Stelleinrichtung beispielsweise ein Antrieb verwendet werden, welcher konstant mit einer Kraft auf das Ausschüttelement einwirkt. Die Stelleinrichtung gibt den Antrieb kurzzeitig frei, so dass die vom Antrieb erzeugte Kraft das Ausschüttelement ein Stück bewegen kann und dadurch eine Teildosis ausgeschüttet wird. Vorzugsweise blockiert die Stelleinrichtung in der Sperrstellung den Antrieb über das Ausschüttelement indem die Stelleinrichtung das Ausschüttelement oder zumindest Teile des Ausschüttelements an einer Bewegung hindert, auch wenn das Ausschüttelement unter Krafteinwirkung steht. In der Freigabestellung ist die Blockierung des Ausschüttelements aufgehoben und die vom Antrieb auf das Ausschüttelement einwirkende Kraft kann das Ausschüttelement oder zumindest Teile des Ausschüttelements bewegen. Je nach Grösse der eingestellten Dosis wird der Vorgang wiederholt bis die gesamte eingestellte Dosis in Teildosen ausgeschüttet ist.

Der Begriff "Medikament" oder "medizinische Substanz" umfasst in diesem Zusammenhang jede fliessfähige medizinische Formulierung, welche geeignet ist zur kontrollierten Verabreichung mittels einer Kanüle oder Hohlnadel, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Unter den Begriffen "Injektionssystem" oder "Injektor" wird in der vorliegenden Beschreibung eine Vorrichtung verstanden, bei der die Injektionsnadel nach Abgabe der medizinischen Substanz aus dem Gewebe des Patienten entfernt wird. Somit verbleibt bei einem Injektionssystem oder bei einem Injektor im Unterschied zu einem Infusionssystem die Injektionsnadel nicht permanent bzw. über einen längeren Zeitraum von mehreren Stunden im Patienten.

Injektoren umfassen üblicherweise eine längliche Kartusche oder Karpule, in der sich die Substanz zum Ausschütten befindet. Diese Kartusche definiert durch ihre längliche Form eine Längsachse. Unabhängig von der Form des Injektors und der Ausrichtung der Kartusche im Injektor, wird in der vorliegenden Beschreibung eine Richtung parallel zu dieser Längsachse der Kartusche als axiale Richtung bezeichnet, während eine Richtung rechtwinklig zur Längsachse der Kartusche als radiale Richtung benannt wird.

Ferner wird ein einstechseitiges Ende des Injektors, an dem sich üblicherweise eine Einstechnadel oder Kanüle befindet, als distales Ende bezeichnet. Entsprechend bezieht sich die Bezeichnung proximales Ende auf ein dem distalen Ende gegenüberliegendes Ende des Injektors.

Unter dem Begriff "Formschluss" wird eine Verbindung verstanden, bei der mindestens zwei Verbindungspartner durch ihre Form ineinandergreifen, so dass eine Bewegung der Verbindungspartner relativ zueinander nicht möglich ist, und infolgedessen Kräfte von einem auf den anderen Verbindungspartner übertragen werden können. Konkret bedeutet das, dass in der Sperrstellung der Antrieb nicht nur angehalten ist, sondern durch ein Ineinandergreifen von Formen in der Stelleinrichtung blockiert ist.

Bevorzugt ist die Stelleinrichtung zum Ausschütten einer eingestellten Dosis mindestens zweimal von der Sperrstellung in die Freigabestellung und von der Freigabestellung wieder in die Sperrstellung verstellbar. Dadurch kann die eingestellte Dosis portionenweise durch mehrere Hübe in mehreren Teildosen erfolgen.

Vorzugsweise ist der Antrieb ein vorgespanntes elastisches Element. Durch die Vorspannung wirkt das elastische Element anhaltend auf das Ausschüttelement ein. Dabei kann eine Kraft und/oder ein Drehmoment direkt oder mittelbar auf das Ausschüttelement wirken. Jedoch kann das elastische Element das Ausschüttelement nur zum Ausschütten bewegen, wenn sich die Stelleinrichtung in der Freigabestellung befindet. Wird die Stelleinrichtung in die Sperrstellung verstellt, sind der Antrieb und vorzugsweise auch das Ausschüttelement blockiert und können sich nicht bewegen. Eine weitere Ausschüttung der Substanz ist dadurch nicht möglich.

Das elastische Element kann eine vorgespannte mechanische Feder, insbesondere eine Druckfeder oder eine Spiralfeder, ein elastisches Kunststoffelement wie ein Elastomerelement oder ein sonstiges Element aus einem elastischen Material, z.B. auch eine Gasfeder sein.

Alternativ besteht die Möglichkeit, dass der Antrieb kein elastisches Element aufweist, sondern beispielsweise durch einen Elektromotor realisiert ist.

Bevorzugt beinhaltet das elastische Element die zur gesamten Ausschüttung der medizinischen Substanz benötigte Energie. Das bedeutet, mit dem vorgespannten elastischen Element kann der gesamte Inhalt der Kartusche, üblicherweise in mehreren Dosen, ausgeschüttet werden. Das elastische Element muss somit während dem Gebrauch einer Kartusche nicht vorgespannt oder aufgezogen werden.

In einer bevorzugten Ausführung ist die Stelleinrichtung als Malteserkreuzgetriebe, welches ein Maltesernockenrad und ein vom Maltesernockenrad getriebenes Maltesernutenrad umfasst, ausgebildet. Das Maltesernockenrad umfasst vorzugsweise einen exzentrisch angeordneten Nocken, während das Maltesernutenrad bevorzugt vier radial ausgerichtete Nuten aufweist. Das Maltesernutenrad kann jedoch auch nur drei oder aber mehr als vier Nuten aufweisen. Entgegen der üblichen Bezeichnung "Malteserkreuzgetriebe" muss somit das Maltesernutenrad nicht zwingend als Kreuz mit vier Nuten ausgebildet sein. In der Freigabestellung greift der Nocken in die Nuten ein, wodurch bei einer Drehung das Maltesernockenrad mit dem Maltesernutenrad zusammenwirkt.

Diese Drehung ist vorzugsweise nur über einen bestimmten Drehwinkel möglich, in dem das Maltesernutenrad nicht durch ein Halteelement des Maltesernockenrades blockiert ist. Solange sich der Nocken des Maltesernockenrades in einer Nut des Maltesernutenrades befindet und das Maltesernutenrad nicht blockiert ist, befindet sich die Stelleinrichtung in der Freigabestellung.

Hat sich das Maltesernutenrad um diesen bestimmten Drehwinkel gedreht, wird es bevorzugt wieder durch das Halteelement des Maltesernockenrads blockiert und der Nocken verlässt die Nut des Maltesernutenrades. Die Stelleinrichtung befindet sich dann in der Sperrstellung und das Maltesernutenrad ist formschlüssig durch den Schaft des Maltesernockenrads gehalten.

Das Malteserkreuzgetriebe ermöglicht somit eine kontrollierte und definierte Drehung des Maltesernutenrads und damit eine kontrollierte, stückweise Bewegung des Ausschüttelements. Das Ausschüttelement wird somit nicht wie aus dem Stand der Technik bekannt, gegen einen Anschlag gestossen, sondern es wird geführt ein Stück weit bewegt, so das eine exakt definierte Menge der Substanz aus der Kartusche ausgestossen wird.

In einer alternativen Ausführung kann die Stelleinrichtung anstelle eines Malteserkreuzgetriebes ein anderes Rastgetriebe umfassen. So kann beispielsweise die Stelleinrichtung ein stufenförmiges Element aufweisen, auf das in der Sperrstellung der Antrieb anhaltend mit einer Kraft auf eine Stufe einwirkt ohne jedoch das Ausschüttelement bewegen zu können. Um eine Teildosis auszuschütten, kann vorzugsweise mit einem Aktuator der Stelleinrichtung das stufenförmige Element bewegt werden, so dass es um eine Stufe verschoben wird, wodurch der Antrieb kurzzeitig freigegeben wird und das Ausschüttelement um die Höhe dieser Stufe bewegen kann.

Während dieser Bewegung in der sich die Stelleinrichtung in der Freigabestellung befindet, kann eine definierte Menge der Substanz aus der Kartusche ausgeschüttet werden. Im Unterschied zum Malteserkreuzgetriebe erfolgt jedoch die Bewegung in der Freigabestellung nicht geführt durch einen in eine Nut eingreifenden Nocken.

Vorzugsweise umfasst die Dosiervorrichtung eine elektrische Betätigungsvorrichtung, mit welcher die Stelleinrichtung von der Sperrstellung in die Freigabestellung und wieder in die Sperrstellung verstellbar ist. Dadurch muss die Stelleinrichtung nicht von Hand durch Muskelkraft betätigt werden, sondern kann elektrisch verstellt werden. Das ist insbesondere vorteilhaft, wenn der Benutzer nur über eingeschränkte Bewegungsmöglichkeiten verfügt, beispielsweise wegen einer Muskelschwäche oder zittrigen Händen.

Die elektrische Betätigungsvorrichtung kann beispielsweise in Form eines elektromagnetischen Aktors mit einem Elektromagneten und einer Spule ausgebildet sein. Ferner kann die Betätigungsvorrichtung als Piezo-Aktor, Elektromotor oder mit einem ansteuerbaren Memory-Metall (Formgedächtnislegierung) realisiert sein. Vorzugsweise ist die Betätigungsvorrichtung als Schrittmotor ausgebildet.

Vorteilhafterweise ist die elektrische Betätigungsvorrichtung fernsteuerbar. Dadurch kann eine Steuereinrichtung für die Betätigungsvorrichtung in einem externen Gerät ausserhalb der Dosiervorrichtung und des Injektors untergebracht sein. Das kann die Bedienung für den Benutzer vereinfachen, da er nicht gleichzeitig den Injektor festhalten muss und die Dosis am Injektor einstellen muss. Stattdessen kann der Benutzer an einer Fernbedienung die Injektionsparameter einstellen und den Injektionsvorgang auslösen. Er kann dann beispielsweise den Injektor mit einer Hand halten und mit der anderen Hand die Fernbedienung betätigen. Da der Injektor über einen Antrieb verfügt und die Stelleinrichtung vorzugsweise elektrisch betätigt wird, muss der Benutzer nicht mit seiner Muskelkraft den Injektor bedienen und insbesondere nicht die Muskelkraft aufbringen, um die Substanz auszuschütten.

Zudem kann dank der Fernsteuerung die Dosiervorrichtung einfach konstruiert werden. So muss die Dosiervorrichtung und auch der Injektor beispielsweise keine Anzeigeeinheit und keine Benutzereingabeeinheit umfassen, da die Anzeige und Benutzereingabemöglichkeiten an der Fernbedienung realisiert werden können. Das ist insbesondere vorteilhaft, wenn der Injektor als Einweg-Injektor ausgebildet ist und möglichst kostengünstig herstellbar sein soll.

Vorzugsweise ist die elektrische Betätigungsvorrichtung drahtlos steuerbar. Somit kann die Datenübertragung, das heisst die Einstellung der Injektionsparameter und das Auslösen der Injektion, von einer Fernbedienung zum Injektor drahtlos erfolgen. Die Fernbedienung kann ein eigens für die Bedienung des Injektors erstelltes Gerät sein. Die Funktionen der Fernsteuerung können aber auch in einer Software implementiert sein, welche auf einem Computer installiert ist. Da kein Kabel vorhanden ist, wird die Handhabung erleichtert.

In einer bevorzugten Ausführung erfolgt die Kommunikation zwischen Fernsteuerung und elektrischer Betätigungsvorrichtung über eine Bluetooth-Funkverbindung. Alternativ kann die Kommunikation auch über ein anderes drahtloses, lokales Netzwerk erfolgen wie beispielsweise mittels ZigBee, Wi-Fi, WiMAX oder LTE.

In einer bevorzugten Ausführung ist die elektrische Betätigungsvorrichtung durch ein mobiles Benutzerendgerät steuerbar. Das Benutzerendgerät kann beispielsweise ein Smartphone, ein Tabletcomputer, eine Smartwatch oder eine Laptop sein. Vorzugsweise lässt die elektrische Betätigungsvorrichtung nicht nur eine momentane Steuerung durch das Benutzerendgerät zu, sondern ist durch das Benutzerendgerät auch programmierbar. So können beispielsweise basierend auf einem im Benutzerendgerät hinterlegten Therapieplanes Dosen zu bestimmten Tageszeiten vorgängig einprogrammiert werden. Dadurch muss der Benutzer zum Injektionszeitpunkt nur die Injektion auslösen und muss nicht vorgängig die Dosis einstellen. Zudem kann das Benutzerendgerät zum Beispiel anhand eines Therapieplanes dem Benutzer Warnungen anzeigen oder mittels optischen, akustischen oder haptischen Signalen an bevorstehende Injektionen erinnern.

In einer bevorzugten Ausführung ist das Ausschüttelement eine Kolbenstangeneinheit, welche eine Gewindestange und einen Kolben umfasst, wobei die Gewindestange relativ zum Kolben drehbar ist und dadurch den im Gehäuse rotationsfest gelagerten Kolben verschieben kann. Bevorzugt steht der Kolben in Gewindeverbindung mit der Gewindestange und ist somit durch eine Drehbewegung der Gewindestange axial verschiebbar relativ zur Gewindestange und relativ zum Gehäuse, in dem die Kolbenstangeneinheit untergebracht ist. Die Gewindestange ist dabei vorzugsweise axial fest relativ zum Gehäuse. Durch diese axiale Verschiebung des Kolbens wird dieser vorzugsweise in die Kartusche geschoben, wodurch die Substanz aus der Kartusche ausgeschüttet wird.

In einer anderen Ausführung besteht auch die Möglichkeit, dass die Gewindestange unbeweglich mit dem Kolben verbunden ist. In diesem Fall kann beispielsweise die Gewindestange in Gewindeverbindung mit dem Gehäuse stehen. Durch Drehen der Gewindestange relativ zum Gehäuse wird diese zusammen mit dem Kolben in axialer Richtung bewegt und der Kolben wird in axialer Richtung in die Kartusche hineingeschoben.

Alternativ zu den beiden oben beschriebenen Ausführungen besteht aber auch die Möglichkeit, dass die Gewindestange nicht drehbar relativ zum Gehäuse ist, sondern nur axial verschiebbar im Gehäuse gelagert ist. In diesem Fall umfasst die Kolbenstangeneinheit bevorzugt eine relativ zur Gewindestange drehbare, jedoch axial relativ zum Gehäuse unbeweglich angeordnete Antriebshülse, welche in Gewindeverbindung mit der Gewindestange steht. Zum Ausschütten wird die Antriebshülse durch den Antrieb gedreht, wodurch die Gewindestange und der Kolben ohne sich zu drehen axial relativ zur Antriebshülse und zum Gehäuse bewegt werden. Der Kolben wird in axialer Richtung in die Kartusche hineingeschoben.

Sowohl die Ausführungen mit der drehenden Gewindestange wie auch die alternative Ausführung mit nicht drehbarer aber verschiebbarer Gewindestange bieten den Vorteil, dass die Kolbenstangeneinheit kompakt konstruiert werden kann.

Bevorzugt ist in der Sperrstellung der Stelleinrichtung die Gewindestange oder die Antriebshülse blockiert, so dass sie in keine Richtung drehbar ist. Dadurch ist sichergestellt, dass in der Sperrstellung die Substanz nicht ausgeschüttet werden kann.

Falls die Stelleinrichtung als Malteserkreuzgetriebe ausgebildet ist, ist vorzugsweise die Gewindestange rotationsfest mit dem Maltesernutenrad verbunden. Somit ist durch die formschlüssige Blockierung des Maltesernutenrades auch die Gewindestange blockiert und es kann keine Ausschüttung der Substanz erfolgen.

Die Erfindung betrifft weiter einen Injektor mit einer Dosiervorrichtung mit den oben beschriebenen Merkmalen und einem Karpulenhalter zur Aufnahme einer Karpule.

Vorzugsweise ist der Injektor ein Einweg-Injektionspen. Ein Einweg-Injektionspen ist ein Injektor, welcher dazu verwendet wird, die in einer nicht nachfüllbaren und nicht austauschbaren Karpule oder vorgefüllten Spritze befindliche Substanz subkutan zu injizieren. Ist die zur Injektion vorgesehene Menge der Substanz in einem oder in mehreren Injektionsvorgängen injiziert worden, wird der Einweg-Injektionspen entsorgt. Die Karpule oder vorgefüllte Spritze im Einweg-Injektionspen kann nicht ausgetauscht werden. Demgegenüber ist bei einem Mehrweginjektor die Karpule oder vorgefüllte Spritze mit der medizinischen Substanz auswechselbar.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
- Fig. 1: zeigt eine Seitenansicht des erfindungsgemässen Injektors,
- Fig. 2: zeigt eine Explosionsdarstellung mit den Bestandteilen der erfindungsgemässen Dosiervorrichtung,
- Fig. 3: zeigt eine perspektivische Ansicht des Malteserkreuzgetriebes in der Sperrstellung ohne Gehäuse des Injektors, und
- Fig. 4: zeigt eine Ansicht des Malteserkreuzgetriebes in der Freigabestellung ohne Gehäuse des Injektors.

### FIGURENBESCHREIBUNG

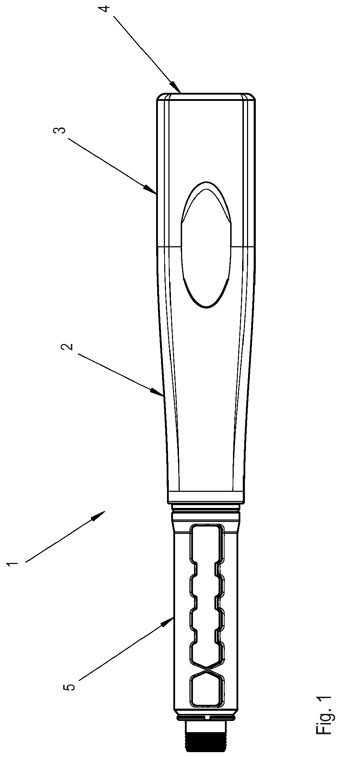

In Figur 1 ist ein Injektor 1 gemäss der Erfindung in der Form eines Einweg-Injektionspens ersichtlich. Der Injektor 1 umfasst ein Gehäuse 2, ein Gehäusehalter 3 und ein Deckel 4. Das Gehäuse 2 ist an seinem distalen Ende mit einem Kartuschenhalter 5 verbunden, in welchem eine Kartusche mit einer medizinischen Substanz aufgenommen ist.

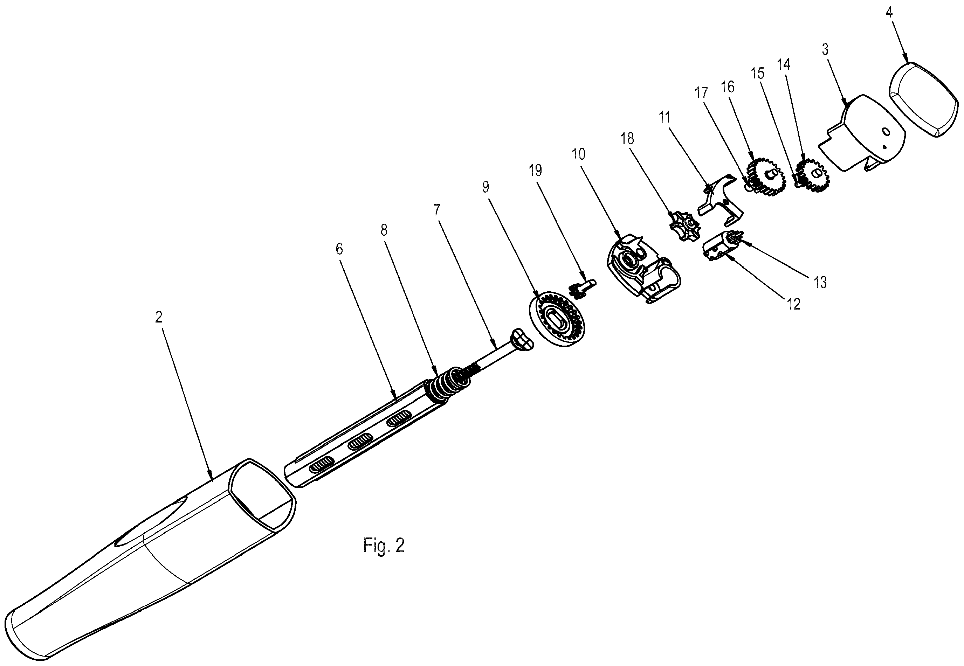

In der Explosionsdarstellung in Figur 2 sind die Einzelteile der erfindungsgemässen Dosiervorrichtung im Injektor 1 ersichtlich. Die Dosiervorrichtung des Injektors 1 beinhaltet eine Getriebeeinheit, eine Kolbenstangeneinheit und eine vorgespannte Antriebsfeder 8 als Antrieb zum Ausschütten der medizinischen Substanz aus dem Injektor 1. Die Kolbenstangeneinheit umfasst eine Gewindestange 7 und einen Kolben 6. Alle Einzelteile sind im Gehäuse 2 untergebracht, welches mit dem Gehäusehalter 3 und dem Deckel 4 verschlossen ist.

Nachfolgend wird anhand der Figur 2 der strukturelle Aufbau der Dosiervorrichtung beschrieben. Anschliessend sind die Funktionen im Detail erläutert.

Die Getriebeeinheit umfasst ein Malteserkreuzgetriebe zum wahlweisen Freigeben oder Sperren des Antriebs des Injektors 1, einen Elektromotor 12 in Form eines Schrittmotors zum Verstellen des Malteserkreuzgetriebes und ein Übersetzungsgetriebe zum Übersetzen des vom Elektromotor 12 erzeugten Drehmoments. Das Malteserkreuzgetriebe umfasst ein Maltesernockenrad 17 mit einem Nocken 17.1 und ein vom Nocken 17.1 angetriebenes Maltesernutenrad 18. Letzteres weist mehrere radial nach aussen verlaufende Nuten auf, in die der Nocken 17.1 eingreifen kann.

Der Elektromotor 12 ist unbeweglich in einer Grundbauplatte 10 gehalten. Er wird mit elektrischer Energie von einer Batterie (nicht dargestellt) versorgt und erzeugt ein Drehmoment, welches von einem Motorenritzel 13 auf ein grösseres erstes Zahnrad 14 übersetzt wird. Das erste Zahnrad 14 ist über eine Welle drehfest mit einem kleineren zweiten Zahnrad 15 verbunden, welches das Drehmoment ein zweites Mal übersetzt, indem dieses mit einem grösseren dritten Zahnrad 16 in Eingriff steht. Das dritte Zahnrad 16 ist auf einer Welle drehfest mit dem Maltesernockenrad 17 verbunden. Dadurch wird das übersetzte Drehmoment weiter auf das Maltesernockenrad 17 übertragen. Der Nocken 17.1 des rotierenden Maltesernockenrades 17 kommt während einer Drehung des Maltesernockenrades 17 abschnittsweise mit dem Maltesernutenrad 18 in Eingriff. Dadurch drehen sich Maltesernockenrad 17 und Maltesernutenrad 18 gleichzeitig und gegenläufig. Über eine Welle ist das Maltesernutenrad 18 drehfest mit einem Ritzel 19 verbunden, welches in ein Innengewinde einer Antriebsscheibe 9 eingreift.

Das erste Zahnrad 14 ist mit dessen Welle und dem zweiten Zahnrad 15 drehbar im Gehäusehalter 3 und der Grundbauplatte 10 gelagert. Ebenso ist das über eine Welle mit dem Maltesernockenrad 17 verbundene dritte Zahnrad 16 im Gehäusehalter 3 und der Grundbauplatte 10 drehbar gelagert. Das Maltesernutenrad 18 ist einerseits in einer Halteplatte 11 drehbar gehalten und andererseits in der Grundbauplatte 10 gelagert. Die Halteplatte 11 stütz sich auf der Grundbauplatte 10 ab. Auch die Antriebsscheibe 9 wird drehbar durch das Gehäuse 2 gelagert.

Die Gewindestange 7 ist unbeweglich mit der Antriebsscheibe 9 verbunden. Zudem steht das Aussengewinde der Gewindestange 7 in Gewindeverbindung mit dem hülsenförmigen Kolben 6, welcher die Gewindestange 7 umgibt und welcher im Gehäuse 2 drehfest, aber axial verschiebbar gelagert ist.

Die vorgespannte Antriebsfeder 8 ist an ihrem ersten Ende stirnseitig an einer Innenseite des hülsenförmigen Kolbens 6 abgestützt und liegt an ihrem zweiten Ende an einem axialen Anschlag im Gehäuse 2 auf.

An einem distalen Ende des Gehäuses 2 weist dieses eine umlaufende Einbuchtung auf, in die der Kartuschenhalter 5 eingeschnappt ist und somit unbeweglich mit dem Gehäuse 2 verbunden ist. Der Kartuschenhalter 5 weist an seinem distalen Ende eine Aufnahme auf, an der eine Injektionskanüle befestigt werden kann. Weiter weist der Kartuschenhalter einen Stopfen auf, auf dem der Kolben 6 aufliegt.

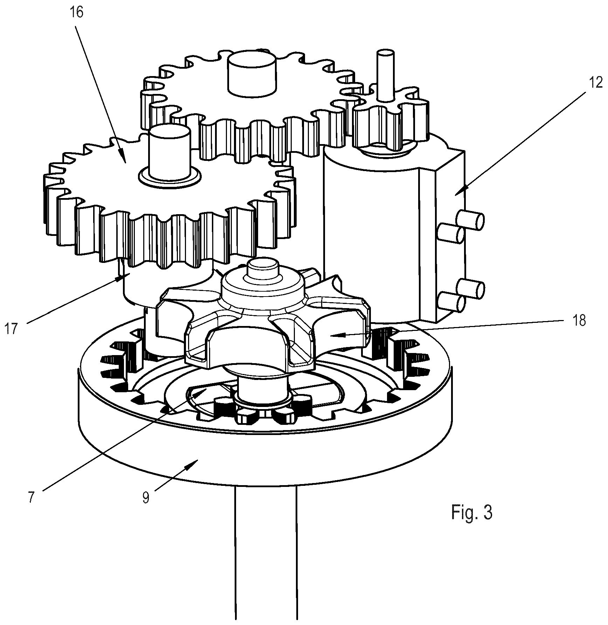

Die Figur 3 zeigt das Malteserkreuzgetriebe in einer Sperrstellung, in der die Antriebsfeder 8 durch den Kolben 6, die Gewindestange und das formschlüssig gehaltene Maltesernutenrad 18 blockiert ist. Durch die Vorspannung der Antriebsfeder 8 wirkt eine Kraft in axialer Richtung auf den Kolben 6. Da der Kolben 6 in Gewindeverbindung mit der Gewindestange 7 steht, wird ein Drehmoment erzeugt, welches von der Gewindestange 7 über die Antriebsscheibe 9 auf das Maltesernutenrad 18 übertragen wird. Dieses kann sich jedoch nicht drehen, da es formschlüssig durch das Maltesernockenrad 17 in Position gehalten wird, insbesondere von einem Segment des Schaftes des Maltesernockenrads, welches in eine entsprechend geformtes, konkaves Gegenstück zwischen zwei Schenkeln des Maltesernutenrads formschlüssig eingreift. Die von der vorgespannten Antriebsfeder 8 erzeugte Kraft wird somit durch den Formschluss im Malteserkreuzgetriebe aufgenommen.

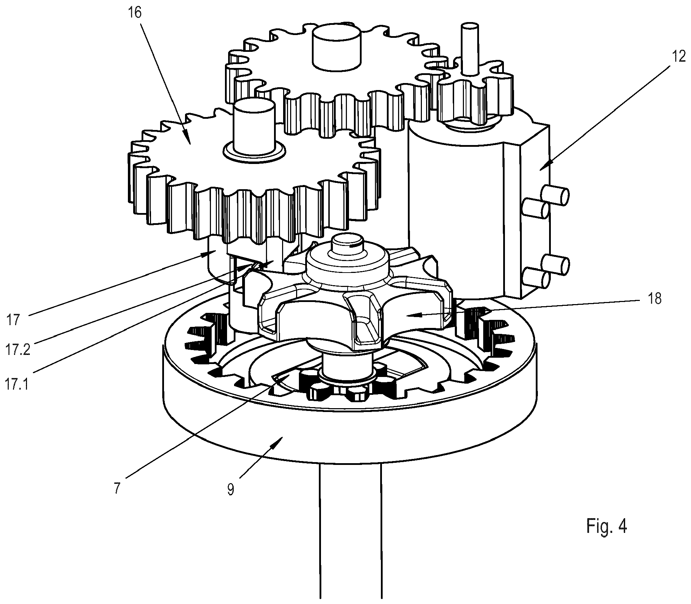

Um eine bestimmte Dosis der medizinischen Substanz auszuschütten, muss das Malteserkreuzgetriebe von dieser Sperrstellung in eine Freigabestellung verstellt werden, welche in Figur 4 ersichtlich ist. Um in die Freigabestellung zu wechseln muss das Maltesernockenrad 17 um seine Achse gedreht werden. Hierzu muss der Elektromotor 12 über das Getriebe das Maltesernockenrad 17 um einen Drehwinkel drehen bis eine Einbuchtung 17.2 im Schaft des Maltesernockenrades 17 den Formschluss zum Maltesernutenrad 18 aufhebt. Gleichzeitig mit der Freigabe des Formschlusses und während das Maltesernockenrad 17 weiter dreht, greift der Nocken 17.1, welcher auf die Einbuchtung 17.2 ausgerichtet ist, in eine radiale Nut in einem der Schenkel im Maltesernutenrad 18 ein und verhindert eine ungebremste Rotation des Maltesernutenrads 18. Maltesernockenrad 17 und Maltesernutenrad 18 drehen sich dann gegenläufig.

Diese Freigabestellung ist solange gegeben, wie der Nocken 17.1 in Eingriff in mit der Nut steht und das Maltesernutenrad 18 bedingt durch die Einbuchtung 17.2 im Schaft des Maltesernockenrades 17 drehbar ist.

Sobald sich das Maltesernockenrad 17 soweit gedreht hat, dass sich die Einbuchtung 17.2 wieder vom Maltesernutenrad 18 weggedreht hat, blockiert der Schaft des Maltesernockenrades 17 das Maltesernutenrad 18. Der Nocken 17.1 steht dann nicht mehr in Eingriff mit der Nut und das Maltesernutenrad 18 ist wieder durch den Schaft des Maltesernockenrades 17 formschlüssig gehalten. Erst nach einer bis auf den Drehwinkel vollständigen Rotation des Maltesernockenrad 17 stehen die Ausbuchtung 17.2 und der Nocken 17.1 wieder für eine Wechselwirkung mit dem Maltesernutenrad 18 zur Verfügung. Der Drehwinkel, während dem das Maltesernockenrad 17 mit dem Maltesernutenrad 18 zusammenwirkt, kann als Einheitsdrehwinkel bezeichnet werden. In der bevorzugten Ausführung entspricht der Einheitsdrehwinkel einer Vierteldrehung des Maltesernutenrades 18. Entsprechend weist dieses vier radiale Nuten und vier konkave Ausnehmungen dazwischen auf. Alternativ oder komplementär zur Übersetzung kann das Maltesernockenrad 17 mehrere über seinen Umfang verteilte Einbuchtungen 17.2 und Nocken 17.1 umfassen. Alternativ kann das Maltesernutenrad 18 auch drei oder fünf Nuten und Ausnehmungen aufweisen.

Während der Drehung um den Einheitsdrehwinkel kann sich die Antriebsfeder 8 ein Stück entspannen, wodurch sich der Kolben 6 in axialer und distaler Richtung bewegt. Das distale Ende des Kolbens 6 wirkt dabei mit dem Stopfen der Kartusche zusammen und stösst den Stopfen etwas in die Kartusche hinein, so dass die medizinische Substanz ausgeschüttet wird.

Der Drehwinkel, um den sich die Gewindestange 7 bei einmaliger Freigabe dreht, bewirkt vorzugsweise eine Ausschüttung von 10 µl der Substanz oder - falls die Substanz ein Insulinpräparat ist - eine Insulineinheit. In einer anderen Ausführung kann diese Menge jedoch auch kleiner oder grösser sein. Die Menge, welche bei einem Einheitsdrehwinkel des Maltesernutenrades 18 während einer Freigabestellung ausgeschüttet wird, ist abhängig von der Übersetzung zwischen dem Maltesernutenrad 18 und dem Kolben 6 und der Grösse des Einheitsdrehwinkels des Malteserkreuzgetriebes. Je nach Grösse der zu verabreichenden Dosis wird das Maltesernockenrad 17 durch den Elektromotor 12 mehrmals um seine Achse gedreht und gibt somit die Antriebsfeder 8 mehrmals frei. Das heisst, das Malteserkreuzgetriebe wechselt mehrmals zwischen der Sperrstellung und der Freigabestellung hin und her (mehrere Einheitsdrehwinkel) um die gesamte Dosis in Teildosen portionenweise auszuschütten. Der Kolben 6 wird dabei stückweise axial verschoben.

Die notwendige Kraft, um den Stopfen in axialer Richtung in die Kartusche zu drücken, wird somit durch die Vorspannung der Antriebsfeder 8 erbracht. Der Elektromotor 12 treibt über das Getriebe nur das Maltesernockenrad 17 an und gibt somit das Maltesernutenrad 18 und die Gewindestange für den bestimmten Drehwinkel während der Freigabestellung frei. Das bedeutet, der Elektromotor 12 erzeugt nicht das Drehmoment für den Vorschub zum Ausschütten, sondern lediglich das Drehmoment zum Verstellen des Malteserkreuzgetriebes.

Der Elektromotor 12 kann mit einer Fernbedienung oder mit einem Benutzerendgerät wie beispielsweise einem Smartphone über Bluetooth angesteuert werden. Die Einstellung der zu verabreichenden Dosis und die Auslösung der Injektion muss somit nicht am Injektor 1 erfolgen, sondern kann am Benutzerendgerät getätigt werden.

### BEZUGSZEICHENLISTE

- 1: Injektor
- 2: Gehäuse
- 3: Gehäusehalter
- 4: Gehäusedeckel

- 5: Kartuschenhalter
- 6: Kolben
- 7: Gewindestange
- 8: Antriebsfeder
- 9: Antriebsscheibe
- 10: Grundbauplatte
- 11: Halteplatte
- 12: Elektromotor

- 13: Motorenritzel
- 14: Erstes Zahnrad
- 15: Zweites Zahnrad
- 16: Drittes Zahnrad
- 17: Maltesernockenrad
- 17.1: Nocken
- 17.2: Einbuchtung
- 18: Maltesernutenrad
- 19: Ritzel

## Patentansprüche

1. Dosiervorrichtung für einen Injektor (1) zur dosierten Abgabe einer medizinischen Substanz umfassend einen Antrieb, ein durch den Antrieb bewegbares Ausschüttelement zum Ausschütten der Substanz aus dem Injektor (1) und eine Stelleinrichtung, welche
a) eine Freigabestellung aufweist, in welcher der Antrieb durch die Stelleinrichtung freigegeben ist und der Antrieb das Ausschüttelement bewegen kann;
b) eine Sperrstellung aufweist, in welcher der Antrieb durch die Stelleinrichtung mittels Formschluss blockiert ist, wodurch der Antrieb das Ausschüttelement nicht bewegen kann,
wobei in der Freigabestellung die Stelleinrichtung mit dem Antrieb gekoppelt ist, so dass eine Bewegung der Stelleinrichtung auf den Antrieb oder eine Bewegung des Antriebs auf die Stelleinrichtung übertragen wird.

2. Dosiervorrichtung nach Anspruch 1, **dadurch gekennzeichnet, dass** die Stelleinrichtung zum Ausschütten einer eingestellten Dosis mindestens zweimal von der Sperrstellung in die Freigabestellung und von der Freigabestellung wieder in die Sperrstellung verstellbar ist.

3. Dosiervorrichtung nach Anspruch 1 oder 2, **dadurch gekennzeichnet, dass** der Antrieb ein vorgespanntes elastisches Element ist.

4. Dosiervorrichtung nach Anspruch 3, **dadurch gekennzeichnet, dass** das elastische Element eine mechanische Feder (8) ist.

5. Dosiervorrichtung nach Anspruch 3 oder 4, **dadurch gekennzeichnet, dass** das elastische Element die zur gesamten Ausschüttung der medizinischen Substanz benötigte Energie beinhaltet.

6. Dosiervorrichtung nach einem der Ansprüche 1 bis 5, **dadurch gekennzeichnet, dass** die Stelleinrichtung ein Malteserkreuzgetriebe ist, umfassend ein Maltesernockenrad (17) und ein mit dem Maltesernockenrad (17) zusammenwirkendes Maltesernutenrad (18).

7. Dosiervorrichtung nach einem der Ansprüche 1 - 6, **dadurch gekennzeichnet, dass** der Injektor (1) eine elektrische Betätigungsvorrichtung umfasst, mit welcher die Stelleinrichtung zwischen der Freigabestellung und der Sperrstellung verstellbar ist.

8. Dosiervorrichtung nach Anspruch 7, **dadurch gekennzeichnet, dass** die elektrische Betätigungsvorrichtung fernsteuerbar ist.

9. Dosiervorrichtung nach Anspruch 8, **dadurch gekennzeichnet, dass** die elektrische Betätigungsvorrichtung drahtlos, insbesondere über Bluetooth, steuerbar ist.

10. Dosiervorrichtung nach Anspruch 8 oder 9, **dadurch gekennzeichnet, dass** die elektrische Betätigungsvorrichtung durch ein mobiles Benutzerendgerät steuerbar ist.

11. Dosiervorrichtung nach einem der Ansprüche 1 - 10, **dadurch gekennzeichnet, dass** das Ausschüttelement eine Kolbenstangeneinheit ist, umfassend eine Gewindestange (7) und einen Kolben (6), wobei die Gewindestange (7) relativ zum Kolben (6) drehbar ist und dadurch den Kolben (6) verschieben kann.

12. Dosiervorrichtung nach Anspruch 11, **dadurch gekennzeichnet, dass** in der Sperrstellung die Gewindestange (7) blockiert ist, so dass sie nicht drehbar ist.

13. Injektor (1) umfassend eine Dosiervorrichtung nach einem der Ansprüche 1 - 12 und einen Karpulenhalter zur Aufnahme einer Karpule.

14. Injektor (1) nach Anspruch 13, **dadurch gekennzeichnet, dass** der Injektor (1) ein Einweg-Injektionspen ist.
